# EUROPEAN PATENT APPLICATION

(11) **EP 2 888 935 A1**
(43) Date of publication of application: **01.07.2015**
(21) Application number: 13830317.7
(22) Date of filing: 21.08.2013
(51) Int. Cl.: A01K 67/00, A23K 1/00, C12Q 1/68, G01N 33/15

(54) **METHOD FOR DETERMINING AUXOTROPHY IN INDUSTRIAL ANIMAL**

(30) Priority: 21.08.2012 JP 2012182206
(71) Applicant: Ajinomoto Co., Inc., Tokyo 104-8315 (JP)
(72) Inventor: TAKIMOTO, Tetsuya, Kawasaki-shi Kanagawa 210-8681 (JP)
(74) Representative: Hoffmann Eitle
(86) International application number: PCT/JP2013/072317
(87) International publication number: WO 2014/030675

(57) **Abstract**

A means for determining the nutritional requirements of an industrial animal is provided. The object can be achieved by identifying an mRNA of which the expression changes before and after change of a physiological condition in an industrial animal by comparing mRNA expression data obtained before and after the change of the physiological condition, identifying a metabolic pathway of which the expression changes before and after the change of the physiological condition on the basis of the identified mRNA, and identifying a metabolite highly required by the industrial animal before or after the change of the physiological condition on the basis of the identified metabolic pathway.

## Description

### Technical Field

The present invention relates to a method for determining the nutritional requirements of an industrial animal, and a feed composition for an industrial animal.

### Background Art

In breeding of industrial animals including mammals, birds, fishes and crustaceans, the development of feeds showing a high growth promoting property is strongly desired. For example, regarding fish, mixed feeds that are currently widely used have been developed on the basis of feeds naturally taken by fish (small fish etc.) as a basic composition. The mixed feeds are produced by, for example, granulating raw materials such as fish meal, fats and oils, cereals, chaff and bran, vitamins, and minerals using a pellet machine or an extruder.

Since it is considered that the nutritional requirements of an industrial animal differ depending on the type and growth stage thereof, it is desirable to develop mixed feeds suitable for the type and growth stage of the industrial animal to be bred.

For example, there are papers discussing the possibility of predicting the nutritional requirements of human on the basis of genome information (Non-patent documents 1 and 2). Moreover, in the field of pet industry, there is known a method for providing a nutritional prescription suitable for an objective pathological condition by obtaining mRNAs from a normal individual of a model animal and an individual showing the pathological condition, comparing the expression amounts thereof, and collating the result with known functional nutrient information (Patent document 1).

However, there is no known method for predicting unknown nutritional requirements specific to the growth stage of an industrial animal.

### Prior art references

### Patent document

Patent document 1: Japanese Patent Laid-open (Kohyo) No. 2010-502198

### Non-patent documents

Non-patent document 1: Tamura T, et al., Genome Inform., 2010 Jan., 22:176-90
Non-patent document 2: Romero P, et al., Genome Biol., 2005;6(1):R2. Epub 2004 Dec 22

### Summary of the Invention

### Object to be Achieved by the Invention

An object of the present invention is to provide a means for determining the nutritional requirements of an industrial animal.

### Means for Achieving the Object

The inventor of the present invention conducted various researches in order to achieve the aforementioned object. As a result, the inventor found that by performing transcriptome analysis for change of the expression of mRNA before and after change of food environment observed in *Fundulus heteroclitus* (mummichog) and mouse, and identifying change of the expression of metabolic pathways on the basis of the result of the analysis, metabolites more highly required before or after the change of the food environment could be identified, and thus accomplished the present invention.

That is, the present invention can be embodied, for example, as follows.
[1] A method for identifying a metabolite highly required by an industrial animal, which comprises:
   identifying an mRNA of which the expression changes before and after change of a physiological condition in the industrial animal by comparing mRNA expression data obtained before and after the change of the physiological condition;
   identifying a metabolic pathway of which the expression changes before and after the change of the physiological condition on the basis of the identified mRNA; and
   identifying a metabolite highly required by the industrial animal before or after the change of the physiological condition on the basis of the identified metabolic pathway.
[2] The method as mentioned above, which comprises obtaining the mRNA expression data.
[3] The method as mentioned above, wherein when the expression of a certain metabolic pathway increases before or after the change of the physiological condition, a metabolite corresponding to upstream of the metabolic pathway and/or a metabolite corresponding to downstream of the metabolic pathway is identified as a metabolite highly required by the industrial animal before or after the change of the physiological condition.
[4] The method as mentioned above, wherein when the expression of a certain metabolic pathway decreases before or after the change of the physiological condition, a metabolite corresponding to downstream of the metabolic pathway and important for growth or life activity, and/or a metabolite corresponding to upstream of the metabolic pathway is identified as a metabolite highly required by the industrial animal before or after the change of the physiological condition.
[5] The method as mentioned above, wherein the change of the physiological condition is selected from ontogenesis, transition in a baby animal between fetal period and a period after the birth, transition in a mother animal between non-gestation period and gestation period, start of feeding behavior, transition in a mother animal between non-lactation period and lactation period, and transition in a baby animal between non-lactation period and lactation period.
[6] A method for producing a feed composition, which comprises:
   identifying a metabolite highly required by an industrial animal before or after change of a physiological condition by the method as mentioned above; and
   adding the metabolite to a raw material of the composition.

### Modes for Carrying out the Invention

### <1> Method of the present invention

The method of the present invention is a method for determining the nutritional requirements of an industrial animal. Specifically, it is a method for identifying a metabolite highly required in an industrial animal.

The method of the present invention comprises (1) identifying an mRNA of which the expression changes before and after change of a physiological condition in the industrial animal by comparing mRNA expression data obtained before and after the change of the physiological condition, (2) identifying a metabolic pathway of which the expression changes before and after the change of the physiological condition on the basis of the identified mRNA, and (3) identifying a metabolite highly required by the industrial animal before or after the change of the physiological condition on the basis of the identified metabolic pathway.

In the present invention, the term "industrial animal" includes domestic animals and fishes for farming.

In the present invention, the term "domestic animals" include, for example, animals for milk, meat, egg, hair, leather, or labor force, and pet animals such as dogs and cats. Specific examples of the "domestic animals" include, for example, cow, pig, chicken, horse, turkey, sheep, goat, dog, and cat. Among these, pig and chicken are preferred. As the chicken, chicken for meat and chicken for egg are preferred, and broilers are more preferred.

In the present invention, the term "fishes for farming" include fishes and crustaceans. Specific examples of the "fishes" include, for example, tuna, bonito, yellowtail, greater amberjack, amberjack, sea bream, salmon, cod, trout, rainbow trout, flatfish, tiger globefish, filefish, horse mackerel, grouper, eel, carp, catfish, tilapia, barramundi, grass carp, silver carp, crucian carp, and *Fundulus heteroclitus* (mummichog). Specific examples of the "crustaceans" include, for example, tiger shrimp, black tiger shrimp, vannamei shrimp, and crabs.

In the present invention, examples of the "change of a physiological condition of an industrial animal" include change of food environment. Examples of the change of food environment include, for example, ontogenesis, transition in a baby animal between fetal period and a period after the birth, transition in a mother animal between non-gestation period and gestation period, start of feeding behavior, transition in a mother animal between non-lactation period and lactation period, and transition in a baby animal between non-lactation period and lactation period (i.e. weaning).

As the mRNA expression data, those opened to public may be used. Examples of the mRNA expression data opened to public include, for example, data registered in publicly known databases and data disclosed in publicly known literature. Specifically, for example, the data registered in the gene expression database GEO of NCBI (http://www.ncbi.nlm.nih.gov/gds) can be used.

Further, as the mRNA expression data, those obtained by conducting gene expression analysis by oneself or outsourcing or the like may also be used. That is, the method of the present invention may comprise obtaining the mRNA expression data for before and after change of a physiological condition of an industrial animal. The gene expression analysis can be conducted by, for example, a known method. Specifically, mRNA expression data for before and after change of a physiological condition of an industrial animal can be obtained by, for example, extracting total RNA from the industrial animal before and after the change of the physiological condition, and performing transcriptome analysis for them.

The mRNA expression data may be those obtained for RNAs extracted from the whole individual of the industrial animal, or may be obtained for RNAs extracted from a partial tissue of the industrial animal. Examples of the partial tissue include, for example, the gastrointestinal tract. Examples of the gastrointestinal tract include, for example, the ileum.

The mRNA expression data may be those obtained for the total mRNAs, or may be those obtained for a part of mRNAs. Examples of a part of mRNAs include, for example, mRNAs corresponding to a specific metabolic pathway. Examples of the specific metabolic pathway include, for example, a metabolic pathway relevant to a specific metabolite. Examples of the "metabolic pathway relevant to a specific metabolite" include a metabolic pathway that generates the specific metabolite, and a metabolic pathway that metabolizes the specific metabolite into another metabolite. Specific examples of the specific metabolite include, for example, saccharides, amino acids, lipids, and vitamins. In the present invention, the "metabolic pathway" may consist of a single reaction step, may consist of two or more sequential reaction steps, or may consist of a combination thereof. The "mRNAs corresponding to a metabolic pathway" refers to one or more kinds of mRNAs from which one or more kinds of proteins that catalyze one or more reaction steps constituting the metabolic pathway are translated.

In the method of the present invention, an mRNA of which the expression changes before and after change of a physiological condition of an industrial animal is identified by comparing mRNA expression data obtained before and after the change of the physiological condition. Hereafter, "mRNA of which the expression changes before and after change of a physiological condition" is also referred to as "expression changing mRNA". Change of the expression is not particularly limited so long as it is a statistically significant change. For statistical analysis, a statistical analysis tool can be used. Specific examples of the statistical analysis tool include, for example, the open source statistical analysis software "R" (http://www.r-project.org/), and the web tool GE02R (http://www.ncbi.nlm.nih.gov/geo/geo2r/). The comparison may be performed for the total mRNAs or for a part of mRNAs. Examples of a part of mRNAs include, for example, mRNAs corresponding to a specific metabolic pathway.

In the method of the present invention, a metabolic pathway of which the expression changes before and after the change of the physiological condition is identified on the basis of the expression changing mRNA identified above. Specifically, the metabolic pathway corresponding to the expression changing mRNA is identified as the metabolic pathway of which the expression changes before and after the change of the physiological condition. The "metabolic pathway corresponding to the expression changing mRNA" refers to a metabolic pathway that is catalyzed by a protein translated from the mRNA. The metabolic pathway corresponding to the expression changing mRNA can be identified on the basis of the function of the protein translated from the mRNA.

The function of a protein can be estimated by comparing the amino acid sequence of the protein with the amino acid sequence(s) of known protein(s). The method of the present invention may comprise estimating the function of a protein, such as mentioned above. The "known protein" is not particularly limited so long as it is a protein or a functional motif of which the amino acid sequence and function are known. The amino acid sequence of a known protein can be an experimentally determined amino acid sequence, or can be an amino acid sequence estimated from genome information or from a gene sequence. The function of a known protein can be an experimentally determined function, or can be a function estimated by comparison with the amino acid sequence(s) of other known protein(s). The function of a protein can be estimated by, for example, comparing the amino acid sequence of the protein with the amino acid sequence(s) of already annotated protein(s) registered at a publicly known database. Comparison of amino acid sequences can be performed by, for example, homology search, motif prediction, or the like. Homology search can be performed by using, for example, BLAST.

The amino acid sequence of a protein can be obtained from, for example, a publicly known database. Although such a database is not particularly limited, examples thereof include, for example, NCBI (http://www.ncbi.nlm.nih.gov/), Ensembl (http://asia.ensembl.org/index.html), DDBJ (DNA Data Bank of Japan, http://www.ddbj.nig.ac.jp/), and EMBL (European Molecular Biology Laboratory, http://www.embl.org/). Specifically, for example, on the ftp site of Ensembl (http://asia.ensembl.org/info/data/ftp/index.html), by selecting "Protein sequence (FASTA)" of an objective biological species, the amino acid sequences of all the proteins of that biological species predicted from the genome information can be downloaded in the FASTA format. Amino acid sequence(s) used in the method of the present invention can also be referred to as "amino acid sequence data".

Amino acid sequence data of a protein can consist of amino acid sequence data deduced from genome information (nucleotide sequence), or can contain other amino acid sequence data. For example, amino acid sequence data of a protein can contain experimentally determined amino acid sequence data.

It is sufficient that amino acid sequence data of a protein is described in a format usable for estimating the function of the protein. The "format usable for estimating the function of a protein" can be appropriately chosen depending on software or an algorithm thereof used for estimating the function of the protein. For example, the amino acid sequence of a protein is preferably described in the FASTA format. The FASTA format is a format containing one or more pieces of sequence data (for example, amino acid sequence data of protein(s)) divided by header line(s). FASTA format including two or more pieces of sequence data is also called multi-FASTA format. The multi-FASTA format is especially useful for annotating two or more kinds of proteins at once. Specifically, for example, when the annotation is performed with KAAS described later, amino acid sequences described in the multi-FASTA format can be batch-processed.

Specifically, for example, amino acid sequence data of a protein described in the FASTA format can be processed on KAAS (KEGG automatic annotation server) of KEGG (Kyoto Encyclopedia of Genes and Genomes, http://www.genome.jp/kegg/) or of iKeg, which is a local server of KEGG, and annotated on the basis of KO (KEGG Orthology), so as to estimate the function of the protein. In such a case, annotation of an objective protein is performed by comparison with the amino acid sequence(s) of known protein(s) to which annotation based on KO on the KEGG database has been added. As for the details of KEGG, for example, Kanehisa M, et al., Nucleic Acids Res., 34, D354-357 (2006), or Kanehisa M, et al., Nucleic Acids Res., 36, D480-D484 (2008) can be referred to. As for the details of KAAS, for example, Moriya Y, et al., Nucleic Acids Res., 35 (Web Server issue):W182-5, Jul. 2007 can be referred to. Processing of amino acid sequence data can be performed for each protein individually, or for two or more kinds of proteins at once. For example, by processing data including the amino acid sequences of two or more kinds of proteins described in the multi-FASTA format on KAAS, annotation of the two or more kinds of proteins based on KO can be performed at once. In the method of the present invention, either one of the estimation of the function of a protein of fish and the estimation of the function of a protein of a reference organism can be performed first, or both of them can be performed simultaneously.

In addition, the method for estimating the function of a protein is not limited to the annotation based on KO, and the function of a protein can be estimated by any method that enables functional classification of a protein based on amino acid sequence. For example, the function of a protein can be estimated by comparison (for example, homology search) with the amino acid sequence(s) of already annotated protein(s) of Swiss-Prot or TrEMBL in UniProtKB (UniProt Knowledgebase, http://www.uniprot.org/help/uniprotkb).

In addition, "estimating the function of a protein by comparing the amino acid sequence of the protein with the amino acid sequence(s) of known protein(s)" includes estimation by processing of arbitrary data that can be converted into the amino acid sequence of the protein, as well as estimation by direct processing of amino acid sequence data of the protein. That is, in the present invention, the function of a protein can be estimated on the basis of arbitrary data that can be converted into the amino acid sequence of the protein. Examples of such data include, for example, the nucleotide sequence of a gene. Such data can be converted into the amino acid sequence of a protein beforehand or at the time of estimation of the function (at the time of annotation), and then can be used.

A metabolic pathway of which the expression changes before and after change of a physiological condition may be identified for all the expression changing mRNAs, or a part of the expression changing mRNAs. For example, such a metabolic pathway may be identified for a certain number of top mRNAs showing largest degrees of the expression change. Although the "certain number of top mRNAs" are not particularly limited, they may be, for example, top 400, top 200, or top 100 mRNAs.

Specifically, a metabolic pathway of which the expression changes before and after change of a physiological condition can be identified by, for example, the PAGE (Parametric Analysis of Gene Set Enrichment) method (Kim and Volsky, BMC Bioinformatics, 2005, 6:144).

The method of the present invention may comprise a step of mapping the functions of proteins translated from expression changing mRNAs on a metabolic pathway. By mapping the functions of proteins translated from expression changing mRNAs on a metabolic pathway, a metabolic pathway of which the expression changes before and after change of a physiological condition can be visualized. Such mapping can be performed by using, for example, the KEGG mapper (http://www.genome.jp/kegg/mapper.html).

In the method of the present invention, on the basis of the above-identified metabolic pathway of which the expression changes before and after change of a physiological condition, a metabolite highly required by an industrial animal before or after the change of the physiological condition is identified. The expression "highly required" means that the metabolite is required in a larger amount after the change of the physiological condition than before the change of the physiological condition, or the metabolite is required in a larger amount before the change of the physiological condition than after the change of the physiological condition.

In an embodiment of the method of the present invention, when the expression of a certain metabolic pathway increases before or after change of a physiological condition, a metabolite corresponding to upstream of the metabolic pathway and/or a metabolite corresponding to downstream of the metabolic pathway may be determined to be a metabolite highly required by the industrial animal before or after the change of the physiological condition. The expression that "the expression of a metabolic pathway increases before change of a physiological condition" means that the degree of the expression of the metabolic pathway before the change of the physiological condition is higher than that observed after the change of the physiological condition. The expression that "the expression of a metabolic pathway increases after change of a physiological condition" means that the degree of the expression of the metabolic pathway after the change of the physiological condition is higher than that observed before the change of the physiological condition.

Further, in an embodiment of the method of the present invention, when the expression of a certain metabolic pathway decreases before or after change of a physiological condition, a metabolite corresponding to upstream of the metabolic pathway may be determined to be a metabolite highly required by the industrial animal before or after the change of the physiological condition. Furthermore, in an embodiment of the method of the present invention, when the expression of a certain metabolic pathway decreases before or after change of physiological condition, a metabolite corresponding to downstream of the metabolic pathway and important for growth or life activity may be identified as a metabolite highly required by the industrial animal before or after the change of the physiological condition. Examples of the metabolite important for growth or life activity include, for example, glucose, aliphatic acids, and amino acids. The expression that "the expression of a metabolic pathway decreases before change of a physiological condition" means that the degree of the expression of the metabolic pathway before the change of the physiological condition is lower than that observed after the change of the physiological condition. The expression that "the expression of a metabolic pathway decreases after change of a physiological condition" means that the degree of the expression of the metabolic pathway after the change of the physiological condition is lower than that observed before the change of the physiological condition.

Examples of "metabolite corresponding to upstream of a metabolic pathway" include a metabolite that serves as the starting substance of the metabolic pathway and a metabolite that is metabolized to thereby generate such a starting substance. Although the range of the upstream varies depending on the expression amount of the metabolic pathway and the presence of a bypass pathway, it may be up to, for example, 20th metabolite, 10th metabolite, 5th metabolite, or third metabolite, on the upstream side from the starting substance of the metabolic pathway as the first metabolite.

Examples of "metabolite corresponding to downstream of a metabolic pathway" include the direct product of the metabolic pathway and a metabolite produced the direct product by further metabolism. Although range of the downstream varies depending on the expression amount of the metabolic pathway and the presence of a bypass pathway, it may be up to, for example, 20th metabolite, 10th metabolite, 5th metabolite, or third metabolite on the downstream side, from the direct product of the metabolic pathway as the first metabolite.

Further, in the present invention, "metabolic pathway" includes an uptake system for a metabolite. The uptake system for a metabolite may be a system that changes the metabolite, or may be a system that does not change the metabolite. Examples of the "metabolite corresponding to upstream of a metabolic pathway" regarding an uptake system for a metabolite include a metabolite that is to be imported by the uptake system. Examples of the "metabolite corresponding to downstream of a metabolic pathway" regarding an uptake system for a metabolite include a metabolite that has been imported by the uptake system and a metabolite that is produced from the imported metabolite by further metabolism.

Whether the nutritional requirements of an industrial animal have been correctly determined by the method of the present invention can be confirmed by feeding the industrial animal with each of feed produced according to the nutritional requirements (for example, feed containing metabolite(s) determined to be highly required by the industrial animal) and control feed (for example, feed not containing the metabolite(s)), and comparing the degrees of growth obtained with the respective feeds.

### <2> Program of the present invention

The program of the present invention is a program for making a computer execute the steps of the method of the present invention.

That is, one embodiment of the program of the present invention is a program for making a computer execute the following steps (1) to (3):
(1) a step of identifying an mRNA of which the expression changes before and after change of a physiological condition in an industrial animal by comparing mRNA expression data obtained before and after the change of the physiological condition,
(2) a step of identifying a metabolic pathway of which the expression changes before and after the change of the physiological condition on the basis of the identified mRNA, and
(3) a step of identifying a metabolite highly required by the industrial animal before or after the change of the physiological condition on the basis of the identified metabolic pathway.

The program of the present invention may make a computer execute a step of estimating the function of a protein of the industrial animal by comparing the amino acid sequence of the protein of the industrial animal with the amino acid sequence of a known protein.

The program of the present invention can be recorded on a recording medium readable by a computer, and provided. A "recording medium readable by a computer" recited herein refers to a recording medium on which information such as data and program can be stored by electric action, magnetic action, optical action, mechanical action, chemical action, or the like, and from which the stored information can be read by a computer. Examples of such a recording medium include, for example, floppy disc (registered trademark), magnetic optical disc, CD-ROM, CD-R/W, DVD-ROM, DVD-R/W, DVD-RAM, DAT, 8 mm tape, memory card, hard disk, ROM (read only memory), SSD, and so forth. As for the program of the present invention, the respective steps to be executed by a computer can be recorded as a single program collectively, or can be recorded as separate programs individually or as an arbitrary combination of separate programs.

### <3> Feed composition of the present invention

A feed composition can be produced by adding a metabolite determined to be highly required by an industrial animal by the method of the present invention to a raw material. That is, the feed composition of the present invention is a feed composition containing a metabolite highly required by an industrial animal before or after change of a physiological condition.

The feed composition of the present invention may consist only of the highly required metabolite identified above, or may further contain another component.

The "other component" is not particularly limited, so long as it is a substance that can be orally ingested by the industrial animal, and for example, those blended and used to feeds or drugs can be used. That is, in an embodiment of the present invention, the feed composition of the present invention can be produced in the same manner by using the same raw materials as those for usual feeds for an industrial animal, except that the highly required metabolite identified above is added to the raw material.

For example, unless the effect of the present invention is degraded, one or more kinds of feed raw materials can be blended to the feed composition of the present invention. Examples of the feed raw material include, for example, brans such as wheat bran, rice bran, barley bran, and millet bran; production lees such as tofu lees, starch lees, copra meal, sake lees, soy sauce lees, brewer's lees, shochu lees, and fruit or vegetable juice lees; cereals such as corn, rice, wheat, barley, and oat; oil meals such as soybean meal, rapeseed meal, cottonseed meal, linseed meal, sesame meal, and sunflower seed meal; animal material feeds such as fish meal, casein, skim milk powder, dried whey, meat bone meal, meat meal, feather meal, and powdered blood; and leaf meals such as alfalfa meal.

Further, for example, unless the effect of the present invention is degraded, one or more kinds of components selected from excipients, fillers, nutrition reinforcers, feed additives, and so forth can also be blended to the feed composition of the present invention. Examples of the excipients include, for example, cellulose derivatives such as carboxymethylcellulose. Examples of the fillers include, for example, dextrin and starch. Examples of the nutrition reinforcers include, for example, vitamins and minerals. Examples of the feed additives include, for example, enzyme preparations and live cell preparations.

The form of the feed composition of the present invention is not particularly limited, unless the effect of the present invention is degraded. The feed composition of the present invention may be in any form such as powder, granule, liquid, paste, and cube.

The feed composition of the present invention can be preferably used for breeding of an industrial animal.

The feed composition of the present invention may be independently administered to an industrial animal, or may be administered to an industrial animal in combination with other feed. When the feed composition of the present invention is administered in combination with other feed, the feed composition of the present invention and the other feed may be administered simultaneously, or may be administered separately. When the feed composition of the present invention and the other feed are administered simultaneously, for example, the feed composition of the present invention can be added to the other feed, and the mixture can be administered.

The feed composition of the present invention may be administered once a day, or two or more times a day as divided portions. The feed composition of the present invention may also be administered once per several days. Dose of the feed composition of the present invention may be or may not be fixed for such administrations, in terms of the amount of the highly required metabolite identified above.

Time of the administration of the feed composition of the present invention is not particularly limited so long as it is administered before or after change of a physiological condition of an industrial animal. That is, when the feed composition of the present invention contains a metabolite highly required by an industrial animal before change of a physiological condition, it may be administered to the industrial animal before the change of the physiological condition. When the feed composition of the present invention contains a metabolite highly required by an industrial animal after change of a physiological condition, it may be administered to the industrial animal after the change of the physiological condition. The feed composition of the present invention may be continuously administered over the whole of the period before or after change of a physiological condition of an industrial animal, or may be administered during only a part of the period before or after change of physiological condition of an industrial animal. For example, the feed composition of the present invention may be continuously administered until immediately before change of a physiological condition of an industrial animal, or may be continuously administered from immediately after change of a physiological condition of an industrial animal. Further, continuation and discontinuation of the administration of the feed composition of the present invention may be repeated during an arbitrary period before or after change of a physiological condition of an industrial animal. Furthermore, the feed composition of the present invention may be administered only either before or after change of a physiological condition of an industrial animal, or may be administered both before and after change of a physiological condition of an industrial animal.

The feed composition of the present invention may also be used, for example, as any feed selected from mother's milk, milk substitute, pre-initial feed (pre-starter feed), initial feed (starter feed), and fattening term feed. The feed composition of the present invention may also be used, for example, in combination with any feed selected from mother's milk, milk substitute, pre-initial feed (pre-starter feed), initial feed (starter feed), and fattening term feed.

### Examples

The present invention will be more specifically explained with reference to the following examples. However, they should not be construed to limit the present invention in any meanings.

### Example 1: Prediction of nutritional requirements of Fundulus heteroclitus (mummichog) after start of feeding behavior

In this example, genes of which the expression changed before and after start of feeding behavior were detected in mummichog, metabolic pathways in which the genes participate were identified, and thereby candidates for nutrients of which the requirement is increased due to the start of feeding behavior were determined. The procedure and results are shown below.

The mRNA expression data set GSE21372, which is registered in the genetic expression database GEO (http://www.ncbi.nlm.nih.gov/gds) in NCBI and includes the results of microarray expression analysis of the processes of embryogenesis, hatching, and growth after fertilization of mummichog egg, was obtained. The data for the period after hatching included in the obtained data set was divided into two groups, i.e., those for the periods before and after the start of feeding behavior, and statistical analysis of change of mRNA expression amounts observed before and after the start of feeding behavior was conducted by using the free statistical analysis software "R" (http://www.r-project.org/). Enzyme numbers were extracted from the microarray platform used in the test, and assigned to the results of expression change analysis. Among genes to which the enzyme numbers were assigned, top 200 genes showing the highest degree of change of expression amount were extracted.

The enzyme numbers of the extracted genes were mapped on metabolic pathways using the KEGG mapper (http://www.genome.jp/kegg/mapper.html) to thereby visualize the metabolic pathways that characteristically changed after the start of feeding behavior.

Considering the details of the metabolic pathways of which the expression changed, candidates for nutrients highly required after the start of feeding behavior were determined. Specifically, it was judged that, for example, the following metabolites: a metabolite generated via a metabolic pathway of which the expression decreased and important for growth or life activity; a metabolite corresponding to upstream of a metabolic pathway of which the expression increased; and/or a metabolite produced by further metabolism from the metabolite corresponding to upstream of a metabolic pathway of which the expression increased; must be acquired from the outside, and thus were preferably supplied from the outside after the start of feeding behavior. Hence, such metabolites were selected as candidates for highly required nutrients. The candidates for nutrients that are highly required after the start of feeding behavior are shown in Table 1.
[Table 1]

**Table 1**

| nutrients | | pathway | gene name | FCR | EC | GB Acce | FCR (log2) | rank_wad | gene expression after stating the feeding action |
|---|---|---|---|---|---|---|---|---|---|
| betain | ko00260 | Glycine, serine and threonine metabolism | Betaine aldehyde dehydrogenase (EC 1218) (BADH)_1643 | 0.83 | 1216 | CN960758 | -0.66 | 588 | - |
| | ko00260 | Glycine, serine and threonine metabolism | Alcohol dehydrogenase (EC 1111)_6635 | 0.76 | 1111 | CN980373 | -0.40 | 1134 | - |
| peptide | ko04874 | Protein digestion end absorption | Carboxypeptidase B precursor (EC 34172)_6300 | 1.47 | 34.172 | CN984239 | 0.56 | 257 | + |
| protein | ko04914 | Protein digestion and absorption | Elastase-2A precursor (EC 342171.71)_3121 | 1.79 | 3.42171 | EV450494 | 0 84 | 206 | + |
| | ko04974 | Protein digestion and absorption | Trypsin II precursor (EC 34214) (Fragment)_3522 | 0.68 | 3.4214 | EV451754 | -0.55 | 1019 | - |
| | ko04974 | Protein digestion and absorption | Chymotrypsin B (EC 34211) [Contains: Chymotrypsin B chain A; Chymotrypsin B chain B]_4066 | 1.35 | 3.4211 | EV453512 | 0.43 | 1138 | + |
| myo-inositol | ko00562 | Inositol phosphate metabolism | Inositol monophosphatase (EC 31.325) (IMPse) (IMP) (Inositol-1(or 4)-monophosphatase) (Lithium-sensitive myo-inositol monophosphatase A1)_1662 | 2.78 | 31325 | CV822196 | 1.48 | 12 | + |
| | ko00562 | Inositol phosphate metabolism | Phosphatidylinositol 4-kinase beta (EC 27167) (Ptdlna 4-kinase) (PI4Kbeta) (PI4K-beta)_873 | 0.75 | 271.67 | CN983629 | -042 | 862 | - |
| oligo (dl) saccharide | ko04974 | Protein digestion and absorption | Maltase-glucoamylase, intestinal [Includes: Maltase (EC 32120) (Alpha-glucosidase): Glucoamylase (EC 3213) (Glucan 1.4-alpha-glucosidase)]_4715 | 1.65 | 321.20 | EV461792 | 0 72 | 699 | + |

### Example 2: Prediction of nutritional requirements of mouse after ablactation

In this example, genes of which the expression changed before and after ablactation were detected in mouse, metabolic pathways in which the genes participate were identified, and thereby candidates for nutrients of which the requirement is increased due to ablactation were determined. The procedure and results are shown below.

The mRNA expression data set GDS2989, which is registered in the genetic expression database GEO (http://www.ncbi.nlm.nih.gov/gds) in NCBI and includes the results of microarray expression analysis of the mouse ileum up to the 32^{nd} day after the birth, was obtained. The data set was divided into two groups, i.e. those before and after the ablactation, and statistical analysis of change of the mRNA expression amounts observed before and after the ablactation was conducted by using the web tool GE02R (http://www.ncbi.nlm.nih.gov/geo/geo2r/).

Amino acid sequence data of mouse proteins were obtained at once from BioMart of Ensembl (http://www.ensembl.org/biomart/martview/243057aba97fc1845e c768160454a20f). The obtained amino acid sequence data were processed on the KEGG Automatic Annotation Server (KAAS) of iKeg (local server of KEGG), annotation based on KEGG Orthology (KO) was collectively performed for the respective proteins, and assigned to the results of expression change analysis. Among the genes, those of which the expression significantly changed were extracted.

KO numbers of the extracted genes were mapped on metabolic pathways using the KEGG mapper (http://www.genome.jp/kegg/mapper.html) to thereby visualize the metabolic pathways that characteristically changed before and after the ablactation.

By using the free statistical analysis software "R" (http://www.r-project.org/), PAGE analysis (Kim and Volsky, BMC Bioinformatics, 2005;6:144) was performed on the basis of GO terms and KEGG pathway names obtainable from GSEA (http://www.broadinstitute.org/gsea/index.jsp) to extract metabolic pathways of which the expression significantly changed before and after the ablactation. The results obtained by using the GO terms are shown in Table 2, and the results obtained by using the KEGG pathway names are shown in Table 3.
[Table 2]

**Table 2**

| rank | p_page | z_page | Geneset name | GO_ID |
|---|---|---|---|---|
| 1 | 2.13E-09 | -5.98744 | PATTERN_RECOGNITION_RECEPTOR_ACTIV1TY | GO:0008329 |
| 2 | 5.38E-08 | -5.43834 | SOLUTE_SODIUM_SYMPORTER_ACTIVITY | GO:0015370 |
| 3 | 2.37E-07 | -5.18752 | EPITHELIAL_CELL_DIFFERENTIATION | GO:0030655 |
| 4 | 5 53E-07 | -5.00707 | BRUSH BORDER | GO:0005903 |
| 5 | 7.08E-07 | -4.95929 | IMMUNE_SYSTEM_PROCESS | GO:0002376 |
| 6 | 7.43E-07 | 4.949718 | LYSOSOME_ORGANIZATION_AND_BIOGENESIS | GO:0007040 |
| 7 | 8.23E-07 | 4.929809 | RECEPTOR_MEDIATED_ENDOCYTOSIS | GO:0006898 |
| 8 | 1.21E-06 | 4.853702 | VACUOLE | GO:0005773 |
| 9 | 1.34E-06 | -4.83392 | HYDROLASE_ACTIVITY_ACTING_ON_CARBON_NITROGEN_NOT_PEPTIDEBONDSIN_CYCLIC_AMIDINES | GO:0016814 |
| 10 | 1.42E-06 | 4.821735 | VITAMIN_TRANSPORT | GO:0051180 |
| 11 | 1.94E-06 | -4.75968 | CARBOXYLESTERASE_ACTIVITY | GO:0004091 |
| 12 | 2.52E-06 | 4.706365 | LYSOSOMAL_MEMBRANE | GO:0005765 |
| 13 | 3.61E-06 | 4.63281 | VACUOLE_ORGANIZATION_AND_BIOGENESIS | GO:0007033 |
| 14 | 3.80E-06 | -4.8219 | MORPHOGENESIS_OF_AN_EPITHELIUM | GO:0002009 |
| 15 | 3.97E-06 | 4.813015 | OXYGEN_AND_REACTIVE_OXYGEN_SPECIES_METABOLIC_PROCESS | GO:0006800 |
| 16 | 5.61E-06 | 4.540605 | COFACTOR_TRANSPORT | GO:0051181 |
| 17 | 6.34E-06 | 4.514574 | HYDROLASE_ACTIVITY_HYDROLYZING_O_GLYCOSYL_COMPOUNDS | GO:0004553 |
| 18 | 7.72E-06 | -4.47277 | ANION_CHANNEL_ACTIVITY | GO:0005253 |
| 19 | 7.72E-06 | -4.47277 | CHLORIDE_CHANNEL_ACTIVITY | GO:0005254 |
| 20 | 7.85E-06 | -4.46913 | IMMUNE_RESPONSE | GO:0006955 |
| 21 | 8.08E-06 | 4.462994 | VlTAMIN_BINDING | GO:0019842 |
| 22 | 8.76E-06 | -4.444564 | DEAMINASE_ACTIVITY | GO:0019239 |
| 23 | 9.93E-06 | -4.41819 | PHOSPHOLIPASE_ACTIVITY | GO:0004620 |
| 24 | 1 03E-05 | 4.411518 | VACUOLAR_MEMBRANE | GO:0005774 |
| 25 | 1.11E-05 | 4394252 | LYSOSOME | GO:0005764 |
| 26 | 1.11E-05 | 4.394252 | LYRIC_VACUOLE | GO:0000323 |
| 27 | 1.30E-05 | -4.36039 | METALLOEXOPEPTIDASE_ACTIVITY | GO:0006235 |
| 28 | 1.31E-05 | 4.359225 | COPPER_ION_BINDING | GO:0005507 |
| 29 | 1.43E-05 | -4.33945 | SERINE_TYPE_ENDOPEPTIDASE_ACTIVITY | GO:0004252 |
| 30 | 1.58E-05 | -4.31674 | TRANSFERASE_ACTIVITY_TRANSFERRING_HEXOSYL_GROUPS | GO:0016756 |
| 31 | 1.71E-05 | -4.29949 | OXIDOREDUCTASE_ACTIVITY_ACTING_ON_SULFUR_GROUP_OF_DONORS | GO:0016667 |
| 32 | 1.98E-05 | -4.26731 | POSITIVI_REGULATION_OF_CELL_ADHESION | GO:0045786 |
| 33 | 2.44E-05 | -4.22041 | PHOSPHOLIPASE_A2_ACTIVITY | GO:0004623 |
| 34 | 3.11E-05 | -4.1663 | LEUKOCYTE_CHEMOTAXIS | GO:0030595 |
| 35 | 526E-05 | -4.04385 | RESPONSE_TO_OTHER_ORGANISM | GO:0051707 |
| 36 | 6.36E-05 | 3.99909 | HYDROLASE_ACTIVITY_ACTING_ON_GLYCOSYL_BONDS | GO:0018799 |
| 37 | 6.58E-05 | -3.9916 | RESPONSE_TO_BIOTIC_STIMULUS | GO:0009607 |
| 38 | 7.59E-05 | 3.95701 | VACUOLAR_PART | GO:0044437 |
| 39 | 7.77E-05 | -3.95151 | POSITIVE_REGULATION_OF_TRANSLATION | GO:0045727 |
| 40 | 8.81E-05 | -3.92127 | TRANSMEMBRANE TRANSPORTER ACTIVITY | GO:0022A51 |

[Table 3]

**Table 3**

| rank | p_page | z_page | Geneset name |
|---|---|---|---|
| 1 | 0 | 12.784 | KEGG_LYSOSOME |
| 2 | 3.55E-15 | 7.8676 | KEGG_OTHER_GLYCAN_DEGRADATION |
| 3 | 4.70E-08 | -5.462 | KEGG_SULFUR_METABOLISM |
| 4 | 5.93E-08 | -5.421 | KEGG_UNOLEIC_ACID_METABOUSM |
| 5 | 7.54E-08 | -5.378 | KEGG_HEMATOPOIETIC_CELL_LINEAGE |
| 6 | 1.68E-06 | 4.7888 | KEGG_GLYCOSPHINGOLIPID_BIOSYNTHESIS_GLOBO_SERIES |
| 7 | 1.92E-06 | -4.762 | KEGG_PRIMARY_IMMUNODEFICIENCY |
| 8 | 3.01E-06 | -4.67 | KEGG_TAURINE_AND_HYPOTAURINE_METABOLISM |
| 9 | 1.72E-05 | -4.299 | KEGG_ALPHA_LINOLENIC_ACID_METABOLISM |
| 10 | 1.81E-05 | -4.287 | KEGG_TOLL_LIKE_RECEPTOR_SIGNALING_PATHWAY |
| 11 | 8.26E-05 | -3.937 | KEGG_CYTOSOUC_DNA_SENSING_PATHWAY |
| 12 | 0.00012 | -3.852 | KEGG_LEISHMANIA_INFECTION |
| 13 | 0.00013 | -3.82 | KEGG_INTESTINAL_IMMUNE_NETWORK_FOR_IGA_PRODUCTION |
| 14 | 0.00015 | 3.7939 | KEGG_GALACTOSE_METABOLISM |
| 15 | 0.00017 | -3.755 | KEGG_GLYCEROPHOSPHOUPID_METABOLISM |
| 16 | 0.00017 | 3.7546 | KEGG_GLYCOSPHINGOLIPID_BIOSYNTHESIS_GANGLIO_SERIES |
| 17 | 0.00027 | -3.641 | KEGQ_GLYCOSPHINGOUPID_BIOSYNTHESIS_LACTO_AND_NEOLACTO_SE |
| 18 | 0.00035 | 3.5749 | KEGO_PENTOSE_AND_GLUCURONATE_INTERCONVERSIONS |
| 19 | 0.00036 | -3.57 | KEGG_FQ_EPSILON_RI_SIGNALING_PATHWAY |
| 20 | 0.00038 | -3.557 | KEGG_CHEMOKINE_SIGNALING_PATHWAY |
| 21 | 0.00056 | -3.452 | KEGG_GNRH_SIGNALING_PATHWAY |
| 22 | 0.00058 | -3.439 | KEGG_MAPK_SIGNALING_PATHWAY |
| 23 | 0.00077 | 3.3622 | KEGG_GLYCOSAMINOGLYCAN_DEGRADATION |

Considering the details of the metabolic pathways, candidates for nutrients highly required after the ablactation were determined on the basis of the results of the PAGE analysis. Specifically, it was judged that, for example, the following metabolites: a metabolite corresponding to downstream of a metabolic pathway of which the expression decreased and important for growth or life activity; a metabolite corresponding to upstream of a metabolic pathway of which the expression decreased; a metabolite corresponding to upstream or downstream of a metabolic pathway of which the expression increased; and/or a metabolite produced by further metabolism from the metabolite corresponding to upstream or downstream of a metabolic pathway of which the expression increased; must be acquired from the outside, and thus were preferably supplied from the outside after the ablactation. Hence, such metabolites were selected as candidates for highly required nutrients. The candidates for nutrients that are highly required after the ablactation are shown in Table 4.
[Table 4]

**Table 4**

| nutrient | | pathway | rank | microarray platform ID | KO | Gene.symbol | Gene.title | adj.P.Val | logFC | the variation of gene expression after first feeding |
|---|---|---|---|---|---|---|---|---|---|---|
| fatty acid | ko00061 | fatty acid biosynthesis | 6349 | 1434185_at | K11262 | Acaca | acetyl-Coenzyme A carboxylase alpha | 5.34E-05 | 0.17215953 | + |
| | ko00061 | fatty acid biosyntesis | 1894 | 1427052_at | NA | LOC100047358/ //Acacb | similar to acetyl-Coenzyme A carboxylase beta///acetyl-Coenxyme A carboxylase beta | 408E-08 | 0.25638442 | + |
| | ko00061 | fatty acid biosynthesis | 5051 | 1423828_at | K00665 | Fasn | fatty acid synthase | 1.18E-05 | 0.2171541 | + |
| | ko00071 | Fatty acid metabolism | 8002 | 1448987_at | K00255 | Acadl | acyl-Coenzyme A dehydrogenase, long-chain | 2.30E-04 | -0.09403258 | - |
| glocuse | ko00010 | Glycolis / Gluconeogenesis | 495 | 1422612_at | K00844 | Hk2 | hexokinase 2 | 262E-11 | 0.59782069 | + |
| | ko00010 | Glycolysis / Gluconeogenesis | 859 | 1416069_at | K00850 | Pfkp | phosphofructokinase, platelet | 486E-10 | 020646377 | + |
| | ko00010 | Glycolysis / Gluconeogenesis | 583 | 1448470_at | K03841 | Fbp1 | fruotose biophosphatase 1 | 654E-11 | -0.62839697 | - |
| | ko00010 | Glycolysis / Gluconeogenesis | 2054 | 1417308_at | K00873 | Pkm2 | pyruvate kinase, muscle | 691E-08 | 0.17880441 | + |

As described above, by estimating metabolic change on the basis of transcriptome information, nutritional requirements specifically observed after change of a physiological condition could be determined in fish and mammal. Further, nutritional requirements specifically observed before change of a physiological condition can also be determined in a similar manner.

### Industrial Applicability

According to the present invention, the nutritional requirements of an industrial animal can be determined. Therefore, mixed feed suitable for the nutritional requirement of an industrial animal can be provided by the present invention.

## Claims

1. A method for identifying a metabolite highly required by an industrial animal, which comprises:
identifying an mRNA of which the expression changes before and after change of a physiological condition in the industrial animal by comparing mRNA expression data obtained before and after the change of the physiological condition;
identifying a metabolic pathway of which the expression changes before and after the change of the physiological condition on the basis of the identified mRNA; and
identifying a metabolite highly required by the industrial animal before or after the change of the physiological condition on the basis of the identified metabolic pathway.

2. The method according to claim 1, which comprises obtaining the mRNA expression data.

3. The method according to claim 1 or 2, wherein when the expression of a certain metabolic pathway increases before or after the change of the physiological condition, a metabolite corresponding to upstream of the metabolic pathway and/or a metabolite corresponding to downstream of the metabolic pathway is identified as a metabolite highly required by the industrial animal before or after the change of the physiological condition.

4. The method according to any one of claims 1 to 3, wherein when the expression of a certain metabolic pathway decreases before or after the change of the physiological condition, a metabolite corresponding to downstream of the metabolic pathway and important for growth or life activity, and/or a metabolite corresponding to upstream of the metabolic pathway is identified as a metabolite highly required by the industrial animal before or after the change of the physiological condition.

5. The method according to any one of claims 1 to 4, wherein the change of the physiological condition is selected from ontogenesis, transition in a baby animal between fetal period and a period after the birth, transition in a mother animal between non-gestation period and gestation period, start of feeding behavior, transition in a mother animal between non-lactation period and lactation period, and transition in a baby animal between non-lactation period and lactation period.

6. A method for producing a feed composition, which comprises:
identifying a metabolite highly required by an industrial animal before or after change of a physiological condition by the method according to any one of claims 1 to 5; and
adding the metabolite to a raw material of the composition.
